# EUROPEAN PATENT APPLICATION

(11) **EP 4 793 346 A1**
(43) Date of publication of application: **19.08.2026**
(21) Application number: 24941231.3
(22) Date of filing: 25.06.2024
(51) Int. Cl.: C12N 9/10, C12N 15/54, C12P 19/56

(54) **GLYCOSYLTRANSFERASE UGT76G1 AND UGT91C1 MUTANTS AND METHOD FOR CATALYTIC SYNTHESIS OF REBAUDIOSIDE A, D, AND M BY USING SAME**

(30) Priority: 29.05.2024 CN 202410682554; 29.05.2024 CN 202410682592; 29.05.2024 CN 202410682588
(71) Applicant: Dongtai Haorui Biotechnology Co., Ltd, Yancheng, Jiangsu 224237 (CN)
(72) Inventor: ZHU, Liping, Weifang, Shandong 262218 (CN); TANG, Shuangyan, Weifang, Shandong 262218 (CN); XU, Liangping, Weifang, Shandong 262218 (CN); SONG, Weicai, Weifang, Shandong 262218 (CN); MA, Xu, Weifang, Shandong 262218 (CN); LIANG, Chaoning, Weifang, Shandong 262218 (CN); LI, Shizhong, Weifang, Shandong 262218 (CN); FENG, Xinyi, Weifang, Shandong 262218 (CN); QIU, Chongshun, Weifang, Shandong 262218 (CN); WANG, Luming, Weifang, Shandong 262218 (CN); JI, Xin, Weifang, Shandong 262218 (CN); BU, Ke, Weifang, Shandong 262218 (CN); LEI, Yanyan, Weifang, Shandong 262218 (CN); ZHU, Lin, Weifang, Shandong 262218 (CN); PENG, Bingbing, Weifang, Shandong 262218 (CN); HOU, Jie, Weifang, Shandong 262218 (CN)
(74) Representative: Metida
(86) International application number: PCT/CN2024/101455
(87) International publication number: WO 2025/245947

(57) **Abstract**

Provided are glycosyltransferase UGT76G1 and UGT91C1 mutants, and methods for synthesizing rebaudiosides A, D, and M under catalysis of the glycosyltransferase UGT76G1 and UGT91C1 mutants, which belong to the technical field of biocatalytic synthesis. The glycosyltransferase UGT76G1 mutant provided in this application can efficiently synthesize the rebaudioside A (RA) with stevioside (STV) as a substrate. The glycosyltransferase UGT91C1 mutant provided in this application is produced through mutation screening based on a wild-type glycosyltransferase UGT91C1, and exhibits a high enzymatic activity and catalytic rate. This glycosyltransferase UGT91C1 mutant can be used to efficiently synthesize the rebaudioside D (RD) with the RA as a substrate. The glycosyltransferase UGT76G1 mutant in this application can also be produced through mutation screening based on a glycosyltransferase UGT76G1, and exhibits a high catalytic activity. This glycosyltransferase UGT76G1 mutant can be used to efficiently synthesize the rebaudioside M (RM) with the RD and uridine diphosphate glucose (UDPG) as substrates.

## Description

### CROSS REFERENCE TO RELATED APPLICATIONS

The present application claims priority to the Chinese Patent Application No. 202410682554.8 filed to the China National Intellectual Property Administration (CNIPA) on May 29, 2024 and entitled "GLYCOSYLTRANSFERASE UGT76G1 MUTANT, AND METHOD FOR SYNTHESIZING REBAUDIOSIDE A (RA) UNDER CATALYSIS OF GLYCOSYLTRANSFERASE UGT76G1 MUTANT", the Chinese Patent Application No. 202410682592.3 filed to the China National Intellectual Property Administration (CNIPA) on May 29, 2024 and entitled "GLYCOSYLTRANSFERASE UGT91C1 MUTANT, AND METHOD FOR SYNTHESIZING REBAUDIOSIDE D (RD) UNDER CATALYSIS OF GLYCOSYLTRANSFERASE UGT91C1 MUTANT", and the Chinese Patent Application No. 202410682588.7 filed to the China National Intellectual Property Administration (CNIPA) on May 29, 2024 and entitled "GLYCOSYLTRANSFERASE UGT76G1 MUTANT, AND METHOD FOR SYNTHESIZING REBAUDIOSIDE M (RM) UNDER CATALYSIS OF GLYCOSYLTRANSFERASE UGT76G1 MUTANT", which are incorporated herein by reference in their entireties.

### TECHNICAL FIELD

The present disclosure relates to the technical field of biocatalytic synthesis, and specifically relates to glycosyltransferase UGT76G1 and UGT91C1 mutants, and methods for synthesizing rebaudiosides A, D, and M under catalysis of the glycosyltransferase UGT76G1 and UGT91C1 mutants.

### BACKGROUND

Rebaudioside A (RA) is a natural non-caloric sweetener extracted from the leaves of the *Stevia rebaudiana* plant. RA does not affect the blood sugar metabolism in the human body, and is considered safe for diabetics. Various international food safety authorities have approved the use of RA as a food additive, recognizing it as safe when used within the specified limits. Compared to other steviol glycosides found in the *Stevia rebaudiana* extract, RA is known for its similar taste to sucrose. RA does not exhibit a noticeable aftertaste of bitterness or metallic taste, and offers a pure and sweet flavor profile. Additionally, RA demonstrates high stability at high temperatures. As a result, RA can be used in thermally processed foods and beverages without undergoing a significant sweetness change or degradation under heat.

RA has exceptionally high sweetness, which is typically considered to be 200 times to 450 times the sweetness of sucrose. Due to the exceptionally high sweetness, only a minimal amount of RA is required to achieve a comparable sweetness level to sucrose, which can remarkably reduce the caloric intake from foods or beverages. Due to low-calorie properties, RA is widely used in the development of various low-sugar, sugar-free, and sugar-reduced products, such as beverages, candies, baked goods, condiments, and table sugars, which can meet the demand of consumers for healthy diets.

Typically, RA is extracted from the leaves of *Stevia rebaudiana* by a separation and purification technique. However, a yield of RA obtained by this technique is relatively low and cannot meet the high market demand.

Rebaudioside D (RD) is a natural, non-nutritive sweetener. RD, as one of the many steviol glycosides present in *Stevia rebaudiana*, is characterized by minimal calories and high sweetness. Compared to other steviol glycosides in the *Stevia rebaudiana* extract, such as RA, RD offers relatively high sweetness with less aftertaste, and provides a similar taste profile to sucrose.

In the food and beverage industries, as a zero-calorie sweetener, RD is extensively used in beverages, baked goods, candies, dairy products, jams, condiments, and a variety of other low-calorie and sugar-free foods to reduce sugar content without compromising sweetness quality. Moreover, since RD does not cause an elevation in a blood sugar level, RD is particularly suitable for diabetics and consumers requiring blood sugar control. Consequently, RD can serve as an ideal sugar substitute. With the increasing attention of consumers on healthy lifestyles, a growing number of sugar-reduced and fat-reduced products adopt RD as a sweetness source to meet the market demand for low-calorie foods.

However, RD is also extracted from the leaves of *Stevia rebaudiana* by a separation and purification technique, and a yield of RD obtained by this technique is relatively low and cannot meet the high market demand.

Rebaudioside M (RM) is a natural sweetener extracted from *Stevia rebaudiana*, and belongs to the steviol glycoside family. In contrast to other common steviol glycosides, such as RA, RM also has a high sweetness level and a relatively improved taste profile close to a taste profile of sucrose, and is nearly zero in calories. Thus, RM is frequently used as an additive in health and weight-loss foods.

Through rigorous food safety evaluations, RM has been recognized as a safe food additive. RM can be used in the food and beverage industries to replace traditional sugars. RM can reduce the calorie content in products without compromising sweetness, and is beneficial for diabetics and individuals requiring weight control to reduce sugar intake. RM can avoid bitterness or lingering tastes to some extent, and thus is used in premium food formulations to optimize taste and flavor profiles.

Nevertheless, the production of RM in the prior art remains relatively low and is insufficient to meet the high market demand.

### SUMMARY

In view of this, an objective of the present disclosure is to provide glycosyltransferase UGT76G1 and UGT91C1 mutants, and methods for synthesizing rebaudiosides A, D, and M under catalysis of the glycosyltransferase UGT76G1 and UGT91C1 mutants. The present disclosure aims to address the issue that the yields of rebaudiosides A, D, and M are relatively low in the prior art.

In a first aspect, the present disclosure provides a glycosyltransferase UGT76G1 mutant, where the glycosyltransferase UGT76G1 mutant is any one selected from the group consisting of the following (A) to (C):
(A) a protein produced through any one or more selected from the group consisting of the following mutations based on an amino acid sequence shown in SEQ ID NO: 1:
   a mutation of a 109th amino acid from L to Q;
   a mutation of a 113th amino acid from S to C; and
   a mutation of a 424th amino acid from I to F;
(B) a protein that has an identity of 95% or more with and the same function as an amino acid sequence defined in the (A); and
(C) a fusion protein produced by linking a tag to a terminus of the protein defined in the (A) or the (B).

Compared with the prior art, the glycosyltransferase UGT76G1 mutant provided in the present disclosure exhibits a higher enzymatic activity than the wild-type glycosyltransferase UGT76G1, and enables the efficient synthesis of RA with stevioside (STV) as a substrate. The optimal mutant 68S can convert 60 mM STV into 60 mM RA within 18, that is, STV can be completely converted into RA. The optimal mutant demonstrates a very promising application prospect in industrial production.

Further, an amino acid sequence of the glycosyltransferase UGT76G1 mutant is shown in SEQ ID NO: 3.

In a second aspect, the present disclosure provides a biological material selected from the group consisting of the following:
(A) a gene encoding the glycosyltransferase UGT76G1 mutant;
(B) a recombinant plasmid carrying the gene in the (A); and
(C) a recombinant cell carrying the recombinant plasmid or the gene encoding the glycosyltransferase UGT76G1 mutant.

The gene is produced through one or more selected from the group consisting of the following mutations based on SEQ ID NO: 2:
a substitution of CTG at positions 325 to 327 in SEQ ID NO: 2 with CAG;
a substitution of AGC at positions 337 to 339 in SEQ ID NO: 2 with TGC; and
a substitution of ATC at positions 1,270 to 1,272 in SEQ ID NO: 2 with TTG.
Preferably, a nucleotide sequence of the gene is shown in SEQ ID NO: 4.

In a third aspect, the present disclosure provides an enzyme composition, including the glycosyltransferase UGT76G1 mutant and sucrose synthase AtSUS,
where the sucrose synthase AtSUS is the following (B1) or (B2): (B1) an amino acid sequence shown in SEQ ID NO: 5; and
(B2) a protein that has an identity of 95% or more with and the same function as the amino acid sequence defined in the (B1).

In a fourth aspect, the present disclosure provides a complete set of recombinant strains expressing the enzyme composition, including a recombinant strain A and a recombinant strain B, where the recombinant strain A includes a recombinant plasmid A, and the recombinant plasmid A is produced by constructing a gene encoding the glycosyltransferase UGT76G1 mutant according to claim 1 or 2 into an expression vector; the recombinant strain B includes a recombinant plasmid B, and the recombinant plasmid B is produced by constructing a gene encoding the sucrose synthase AtSUS into a plasmid; and a nucleotide sequence of the gene encoding the sucrose synthase AtSUS is shown in SEQ ID NO: 6.

Further, host strains include, but are not limited to, *Escherichia coli*, *Saccharomyces cerevisiae*, *Pichia pastoris*, or *Corynebacterium glutamicum*.

In a fifth aspect, the present disclosure provides a method for synthesizing RA under catalysis of a glycosyltransferase UGT76G1 mutant, including the following steps:
adding STV, uridine diphosphate glucose (UDPG), sucrose, and the enzyme composition or inducibly expressed enzyme products of the complete set of recombinant strains to a catalytic reaction system, conducting a reaction, and conducting enzyme inactivation and centrifugation to produce a supernatant including the RA.

Compared with the prior art, the present disclosure utilizes STV, UDPG, and sucrose as reaction substrates and employs a dual-enzyme coupled catalytic system involving the glycosyltransferase UGT76G1 mutant and sucrose synthase AtSUS. This approach not only enables the cyclic regeneration of UDPG as a substrate in the efficient production of the RA, but also allows the indirect measurement of an activity of a glycosyltransferase based on the quantitative analysis of fructose as a by-product, thereby facilitating the selection of a superior mutant with high enzymatic activity.

Further, the inducibly expressed enzyme products of the complete set of recombinant strains include an inducibly expressed enzyme product A and an inducibly expressed enzyme product B;
a process for acquiring the inducibly expressed enzyme product A includes the following steps:
inoculating a seed culture of the recombinant strain A into a medium including kanamycin sulfate, and culturing; when OD₆₀₀ of a resulting culture reaches 0.6 to 0.8, adding L-arabinose, and conducting induction for 8 h to 40 h; conducting centrifugation to produce a first cell pellet, and collecting the first cell pellet; subjecting the first cell pellet to disruption, conducting centrifugation to produce a first supernatant, and collecting the first supernatant, which is the inducibly expressed enzyme product A, where an inoculum size is 1 v/v%, a final concentration of the kanamycin sulfate is 10 µg/mL to 100 µg/mL, and a final concentration of the L-arabinose is 0.1 mM to 15 mM; and
a process for acquiring the inducibly expressed enzyme product B includes the following steps:
   inoculating a seed culture of the recombinant strain B into a medium including kanamycin sulfate, and culturing at 30°C to 40°C and 200 r/min to 300 r/min until OD₆₀₀ of a resulting culture reaches 0.6 to 0.8; adding L-arabinose, and further culturing for 8 h to 40 h; conducting centrifugation to produce a second cell pellet, and collecting the second cell pellet; subjecting the second cell pellet to disruption, conducting centrifugation to produce a second supernatant, and collecting the second supernatant, which is the inducibly expressed enzyme product B, where an inoculum size is 1 v/v%, a final concentration of the kanamycin sulfate is 10 µg/mL to 100 µg/mL, and a final concentration of the L-arabinose is 0.1 mM to 15 mM.

Further, in the catalytic reaction system, a concentration of the STV is 10 mM to 100 mM, a concentration of the UDPG is 0.1 mM to 5 mM, a concentration of the sucrose is 50 mM to 800 mM, an amount of the inducibly expressed enzyme product A of the recombinant strain A added is 0.1 mL to 50 mL, and an amount of the inducibly expressed enzyme product B of the recombinant strain B added is 0.1 mL to 50 mL.

Further, in the catalytic reaction system, a pH is 5.0 to 8.0, a temperature is 25°C to 60°C, and the reaction is conducted for 5 h to 30 h.

In a sixth aspect, the present disclosure provides a glycosyltransferase UGT91C1 mutant, where the glycosyltransferase UGT91C1 mutant is any one selected from the group consisting of the following (A) to (C):
(A) a protein produced through any one or more selected from the group consisting of the following mutations based on an amino acid sequence shown in SEQ ID NO: 20:
   a mutation of a 89th amino acid from N to Y;
   a mutation of a 155th amino acid from M to L;
   a mutation of a 274th amino acid from S to T; and
   a mutation of a 361st amino acid from N to S;
(B) a protein that has an identity of 95% or 98% or more with and the same function as an amino acid sequence defined in the (A); and
(C) a fusion protein produced by linking a tag to a terminus of the protein defined in the (A) or the (B).

Compared with the prior art, the glycosyltransferase UGT91C1 mutant provided in the present disclosure is produced through mutation screening based on a wild-type glycosyltransferase UGT91C1, and exhibits a high enzymatic activity and catalytic rate. The optimal mutant 2-12E can convert 27.4 mM RA into 25.8 mM RD within 15 h with a conversion rate of 94.2% for the RA, which significantly improves the yield of the RD.

Further, an amino acid sequence of the glycosyltransferase UGT91C1 mutant is shown in SEQ ID NO: 22.

In a seventh aspect, the present disclosure provides a biological material including any one selected from the group consisting of the following:
(A) a gene encoding the glycosyltransferase UGT91C1 mutant;
(B) a recombinant plasmid carrying the gene in the (A); and
(C) a recombinant cell carrying the recombinant plasmid or the gene encoding the glycosyltransferase UGT91C1 mutant.

The gene is produced through one or more selected from the group consisting of the following mutations based on SEQ ID NO: 21:
a substitution of AAC at a position 89 in SEQ ID NO: 21 with TAT;
a substitution of ATG at a position 155 in SEQ ID NO: 21 with TTG;
a substitution of TCC at a position 274 in SEQ ID NO: 21 with ACG; and
a substitution of AAC at a position 361 in SEQ ID NO: 21 with AGC.

Preferably, a nucleotide sequence of the gene is shown in SEQ ID NO: 23.

In an eighth aspect, the present disclosure provides an enzyme composition, including the glycosyltransferase UGT91C1 mutant and sucrose synthase AtSUS,
where the sucrose synthase AtSUS is the following (B1) or (B2): (B1) an amino acid sequence shown in SEQ ID NO: 5; and
(B2) a protein that has an identity of 95% or 98% or more with and the same function as the amino acid sequence defined in the (B1).

In a ninth aspect, the present disclosure provides a set of recombinant strains for expressing the enzyme composition, including a recombinant strain A and a recombinant strain B,
where the recombinant strain A includes the recombinant plasmid;
the recombinant strain B includes a recombinant plasmid B, and the recombinant plasmid B is produced by constructing a gene encoding the sucrose synthase AtSUS into a plasmid; and a nucleotide sequence of the gene encoding the sucrose synthase AtSUS is shown in SEQ ID NO: 6.

Further, host strains include, but are not limited to, *Escherichia coli*, *Saccharomyces cerevisiae*, *Pichia pastoris*, or *Corynebacterium glutamicum*.

In a tenth aspect, the present disclosure provides a method for synthesizing RD under catalysis of a glycosyltransferase UGT91C1 mutant, including the following steps:
adding RA, UDPG, sucrose, and the enzyme composition or inducibly expressed enzyme products of the complete set of recombinant strains to a catalytic reaction system, conducting a reaction, and conducting enzyme inactivation and centrifugation to produce a supernatant including the RD.

Compared with the prior art, the glycosyltransferase UGT91C1 mutant can be used for the catalytic synthesis of RD and uridine diphosphate (UDP) through a glycosylation reaction with RA and UDPG as substrates, and the sucrose synthase AtSUS can catalyze the production of UDPG and fructose from UDP and sucrose. Therefore, the present disclosure adopts the RA, UDPG, and sucrose as substrates, and couples the glycosyltransferase UGT91C1 mutant with the sucrose synthase AtSUS in a catalytic reaction. On one hand, this solution enables the indirect measurement of an activity of a glycosyltransferase based on the quantitative analysis of fructose as a by-product, thereby facilitating the selection of a glycosyltransferase UGT91C1 mutant with high catalytic activity. On the other hand, the introduction of the sucrose synthase AtSUS achieves the cyclic regeneration of UDPG and further enhances the yield of the RD.

Further, the inducibly expressed enzyme products of the complete set of recombinant strains include an inducibly expressed enzyme product A and an inducibly expressed enzyme product B;
a process for acquiring the inducibly expressed enzyme product A includes the following steps:
inoculating a seed culture of the recombinant strain A according to claim 5 into a medium including kanamycin sulfate, and culturing; when OD₆₀₀ of a resulting culture reaches 0.6 to 0.8, adding isopropyl-β-D-thiogalactoside (IPTG), and conducting induction for 8 h to 40 h; conducting centrifugation to produce a first cell pellet, and collecting the first cell pellet; subjecting the first cell pellet to disruption with a lysozyme solution or to ultrasonic disruption, conducting centrifugation to produce a first supernatant, and collecting the first supernatant, which is the inducibly expressed enzyme product A, where a final concentration of the kanamycin sulfate is 10 µg/mL to 100 µg/mL and a final concentration of the IPTG is 0.01 mM to 1 mM; and
a process for acquiring the inducibly expressed enzyme product B includes the following steps:
   inoculating a seed culture of the recombinant strain B according to claim 5 into a medium including kanamycin sulfate, and culturing at 25°C to 40°C and 200 r/min to 300 r/min until OD₆₀₀ of a resulting culture reaches 0.6 to 0.8; adding L-arabinose, and conducting induction for 8 h to 40 h; conducting centrifugation to produce a second cell pellet, and collecting the second cell pellet; subjecting the second cell pellet to disruption with a lysozyme solution or to ultrasonic disruption, conducting centrifugation to produce a second supernatant, and collecting the second supernatant, which is the inducibly expressed enzyme product B, where a final concentration of the kanamycin sulfate is 10 µg/mL to 100 µg/mL and a final concentration of the L-arabinose is 0.1 mM to 15 mM.

Further, in the catalytic reaction system, a concentration of the RA is 5 mM to 100 mM, a concentration of the UDPG is 0.1 mM to 5 mM, a concentration of the sucrose is 40 mM to 800 mM, an amount of the inducibly expressed enzyme product A added is 0.1 mL to 50 mL, and an amount of the inducibly expressed enzyme product B added is 0.1 mL to 50 mL.

Further, in the catalytic reaction system, a pH is 5.0 to 8.0, a temperature is 25°C to 60°C, and the reaction is conducted for 5 h to 30 h.

In an eleventh aspect, the present disclosure provides a glycosyltransferase UGT76G1 mutant, where the glycosyltransferase UGT76G1 mutant is any one selected from the group consisting of the following (A) to (C):
(A) a protein produced through any one or more selected from the group consisting of the following mutations based on an amino acid sequence shown in SEQ ID NO: 1:
   a mutation of an 89th amino acid from M to H;
   a mutation of a 380th amino acid from L to M; and
   a mutation of a 411th amino acid from A to Y;
(B) a protein that has an identity of 95% or 98% or more with and the same function as an amino acid sequence defined in the (A); and
(C) a fusion protein produced by linking a tag to a terminus of the protein defined in the (A) or the (B).

Compared with the prior art, the glycosyltransferase UGT76G1 mutant provided in the present disclosure can be produced through mutation screening based on a glycosyltransferase UGT76G1, and exhibits a high catalytic activity. This glycosyltransferase UGT76G1 mutant can be used to efficiently synthesize RM with the RD and UDPG as substrates.

Further, an amino acid sequence of the glycosyltransferase UGT76G1 mutant is shown in SEQ ID NO: 30.

In a twelfth aspect, the present disclosure provides a biological material including any one selected from the group consisting of the following:
(A) a gene encoding the glycosyltransferase UGT76G1 mutant;
(B) a recombinant plasmid carrying the gene in the (A); and
(C) a recombinant cell carrying the recombinant plasmid in the (B) or the gene in the (A).

The gene is produced through one or more selected from the group consisting of the following mutations based on SEQ ID NO: 2:
a substitution of ATG at a position 89 in SEQ ID NO: 2 with CAT;
a substitution of CTC at a position 380 in SEQ ID NO: 2 with ATG; and
a substitution of GCA at a position 411 in SEQ ID NO: 2 with TAT.

Preferably, a nucleotide sequence of the gene is shown in SEQ ID NO: 31.

In a thirteenth aspect, the present disclosure provides an enzyme composition, including the glycosyltransferase UGT76G1 mutant and sucrose synthase AtSUS,
where the sucrose synthase AtSUS is the following (B1) or (B2):
(B1) an amino acid sequence shown in SEQ ID NO: 5; and
(B2) a protein that has an identity of 95% or 98% or more with and the same function as the amino acid sequence defined in the (B1).

In a fourteenth aspect, the present disclosure provides a method for synthesizing RM under catalysis of a glycosyltransferase UGT76G1 mutant, including: with RD, UDPG, and sucrose as substrates, synthesizing the RM under catalysis of the enzyme composition.

Further, in a catalytic reaction system, a concentration of the RD is 5 mM to 100 mM, a concentration of the UDPG is 0.1 mM to 5 mM, a concentration of the sucrose is 40 mM to 800 mM, an amount of a glycosyltransferase UGT76G1 mutant solution added is 0.1 mL to 50 mL, and an amount of a sucrose synthase AtSUS solution added is 0.1 mL to 50 mL.

Further, a preparation process of the glycosyltransferase UGT76G1 mutant solution includes the following steps:
(1) constructing a gene encoding the glycosyltransferase UGT76G1 mutant into an expression vector to produce a recombinant plasmid A, and transforming the recombinant plasmid A into a host strain to produce a recombinant strain A; and
(2) inoculating a seed culture of the recombinant strain A into a medium including kanamycin sulfate, and culturing; when OD₆₀₀ of a resulting culture reaches 0.6 to 0.8, adding L-arabinose, and conducting induction at 25°C to 40°C for 8 h to 40 h; conducting centrifugation, and collecting a resulting cell pellet; subjecting the cell pellet to disruption, and conducting centrifugation to produce a supernatant, which is the glycosyltransferase UGT76G1 mutant solution, where an inoculum size is 1 v/v%, a final concentration of the kanamycin sulfate is 10 µg/mL to 100 µg/mL, and a final concentration of the L-arabinose is 0.1 mM to 15 mM.

Further, a preparation process of the sucrose synthase AtSUS solution includes the following steps:
(1) constructing a gene encoding the sucrose synthase AtSUS into an expression vector to produce a recombinant plasmid B, and transforming the recombinant plasmid B into a host strain to produce a recombinant strain B, where a nucleotide sequence of the gene encoding the sucrose synthase AtSUS is shown in SEQ ID NO: 6; and
(2) inoculating a seed culture of the recombinant strain B into a medium including kanamycin sulfate, and culturing; when OD₆₀₀ of a resulting culture reaches 0.6 to 0.8, adding L-arabinose, and conducting induction at 25°C to 40°C for 8 h to 40 h; conducting centrifugation, and collecting a resulting cell pellet; subjecting the cell pellet to disruption, and conducting centrifugation to produce a supernatant, which is the sucrose synthase AtSUS solution, where an inoculum size is 1 v/v%, a final concentration of the kanamycin sulfate is 10 µg/mL to 100 µg/mL, and a final concentration of the L-arabinose is 0.1 mM to 15 mM.

Further, the host strain includes, but is not limited to, *Escherichia coli*, *Saccharomyces cerevisiae*, *Pichia pastoris*, or *Corynebacterium glutamicum*.

Further, in the catalytic reaction system, a pH is 5.0 to 8.0, a temperature is 25°C to 60°C, and a reaction is conducted for 5 h to 30 h.

Compared with the prior art, the present disclosure adopts a dual-enzyme coupled catalytic system involving a glycosyltransferase UGT76G1 mutant and sucrose synthase AtSUS to achieve the catalytic synthesis of RM with sucrose, UDPG, and RD as substrates. In this process, the cyclic regeneration of UDPG is allowed, which reduces the consumption of the substrate. Moreover, fructose generated during a reaction can be quantitatively analyzed to indirectly measure an activity of the glycosyltransferase UGT76G1 mutant. Based on this characteristic, directed evolution and engineering can be applied to a glycosyltransferase UGT76G1, thereby enhancing the catalytic activity of the glycosyltransferase.

### BRIEF DESCRIPTION OF THE DRAWINGS

FIG. 1 shows yields of RA under catalysis of different glycosyltransferase UGT76G1 mutants in Example 3;
FIG. 2 shows a variation trend of a yield of RA over the conversion time in Example 4;
FIG. 3 shows yields of RD under catalysis of different glycosyltransferase UGT91C1 mutants;
FIG. 4 shows a variation trend of a yield of RD over the conversion time;
FIG. 5 shows yields of RM under catalysis of different glycosyltransferase UGT76G1 mutants; and
FIG. 6 shows a variation trend of a yield of RM over the conversion time.

### DETAILED DESCRIPTION OF THE EMBODIMENTS

To make the technical problems to be solved, the technical solutions, and the beneficial effects of the present disclosure clear, the present disclosure is described in further detail below with reference to specific examples. It should be understood that the specific examples described herein are merely intended to explain the present disclosure, rather than to limit the present disclosure.

### Example 1

### Preparation of a recombinant strain expressing sucrose synthase AtSUS

With Inf-pYB1k-atsus-F and Inf-pYB1k-atsus-R as primers and cDNA of *Arabidopsis thaliana*-derived sucrose synthase AtSUS as a template, polymerase chain reaction (PCR) amplification was carried out using a high-fidelity DNA polymerase (purchased from ABclonal Technology Co., Ltd., Wuhan, China) to produce a correct atsus gene fragment. A nucleotide sequence of a gene encoding the sucrose synthase AtSUS was shown in SEQ ID NO: 6. An amino acid sequence of the sucrose synthase AtSUS was shown in SEQ ID NO: 5.
Inf-pYB1k-atsus-F:
   GCTAACAGGAGGAATTAACCATGGAAAATAAAACGGAGACC (SEQ ID NO: 7).
Inf-pYB1k-atsus-R:
   CCAGATCTACCCTCGAGTTACAACGATGAAATGTAAGAAAC (SEQ ID NO: 8).

With pYB1k-F and pYB1k-R as primers and a pYB1k empty vector as a template, PCR amplification was carried out using a high-fidelity DNA polymerase (purchased from ABclonal Technology Co., Ltd., Wuhan, China) to produce a correct pYB1k expression vector fragment (the pYB1k empty vector had been disclosed in You R, Wang L, Shi C, Chen H, Zhang S, Hu M, Tao Y. Efficient production of myo-inositol in Escherichia coli through metabolic engineering. Microb. Cell Fact. 2020 May 24; 19 (1): 109.).
pYB1k-F: CTCGAGGGTAGATCTGGTAC (SEQ ID NO: 9).
pYB1k-R: GGTTAATTCCTCCTGTTAGC (SEQ ID NO: 10).

The atsus gene fragment and the pYB1k expression vector fragment were ligated by a Gibson assembly method.

A Gibson ligation product was added to DH5α competent *Escherichia coli* cells (purchased from TransGen Biotech Co., Ltd., Beijing, China). A resulting mixture was incubated on ice for 30 min, then incubated in a 42°C water bath for 90 s, and then placed on ice for 2 min. Transformed *Escherichia coli* cells were added to 1 mL of an LB medium, allowed to recover in a 37°C shaker for 1 h, coated on a kanamycin-containing LB agar plate, and cultured overnight at 37°C. A plurality of single colonies were picked and cultured. PCR verification was carried out using primers pBAD-F and atsus-F300-R. Positive clones with a target sequence of a correct size were picked and cultured, and a plasmid was then extracted to obtain a positive-clone plasmid pYB1k-atsus.
pBAD-F: GATTATTTGCACGGCGTCAC (SEQ ID NO: 11).
atsus-F300-R: CTTGTGGGTCGTTGTCGAGGATG (SEQ ID NO: 12).

The plasmid pYB1k-atsus was transformed into BW25113 competent *Escherichia coli* cells. Transformed *Escherichia coli* cells were then coated on a kanamycin-containing LB agar plate and cultured overnight at 37°C. Positive clones carrying the plasmid pYB1k-atsus were selected, which were the recombinant *Escherichia coli* strain expressing the sucrose synthase AtSUS.

### Example 2

Preparation of a recombinant strain expressing a wild-type glycosyltransferase UGT76G1

With Inf-pRB1k-ugt76g1-F and Inf-pRB1k-ugt76g1-R as primers and cDNA of a wild-type *Stevia rebaudiana*-derived glycosyltransferase UGT76G1 as a template, PCR amplification was carried out using a high-fidelity DNA polymerase (purchased from ABclonal Technology Co., Ltd., Wuhan, China) to produce a correct ugt76g1 gene fragment.
Inf-pRB1k-ugt76g1-F:
   CTAACAGGAGGAATTAACCATATGGCCGAAAACAAGACCGA (SEQ ID NO: 13).
Inf-pRB1k-ugt76g1-R:
   GTACCAGATCTACCCTCGAGTTACAAAGAGGAAATGTAAGA (SEQ ID NO: 14).

With pRB1k-XhoI-F and pRB1k-NcoI-R as primers and a pRB1k empty vector as a template, PCR amplification was carried out using a high-fidelity DNA polymerase (purchased from ABclonal Technology Co., Ltd., Wuhan, China) to produce a correct pRB1k expression vector fragment (the pRB1k empty vector had been disclosed in Qun Liu, Baixue Lin, Yong Tao. Improved methylation in E. coli via an efficient methyl supply system driven by betaine. Metabolic Engineering, Volume 72, 2022, 46-55).
pRB1k-XhoI-F: CTCGAGGGTAGATCTGGTAC (SEQ ID NO: 15).
pRB1k-NcoI-R: GGTTAATTCCTCCTGTTAGC (SEQ ID NO: 16).

The ugt76g1 gene fragment and the pRB1k expression vector fragment were ligated by a Gibson assembly method. A Gibson ligation product was added to DH5α competent *Escherichia coli* cells (purchased from TransGen Biotech Co., Ltd., Beijing, China). A resulting mixture was incubated on ice for 30 min, then incubated in a 42°C water bath for 90 s, and then placed on ice for 2 min. Transformed *Escherichia coli* cells were added to 1 mL of an LB medium, allowed to recover in a 37°C shaker for 1 h, coated on a kanamycin-containing LB agar plate, and cultured overnight at 37°C. A plurality of single colonies were picked and cultured. PCR verification was carried out using primers pBAD-F and ugt76g1-F300-R. Positive clones with a target sequence of a correct size were picked and cultured, and a plasmid was then extracted to obtain a positive-clone plasmid pRB1k-ugt76g1.
pBAD-F: GATTATTTGCACGGCGTCAC (SEQ ID NO: 11).
ugt76g1-F300-R:CTGCAGTTCCAGTTCACGAC (SEQ ID NO: 17).

The plasmid pRB1k-ugt76g1 was transformed into BW25113 competent *Escherichia coli* cells. Transformed *Escherichia coli* cells were then coated on a kanamycin-containing LB agar plate and cultured overnight at 37°C. The next day, positive clones carrying the plasmid pRB1k-ugt76g1 were selected, which were the recombinant *Escherichia coli* strain expressing the wild-type glycosyltransferase UGT76G1.

### Example 3

Construction of a glycosyltransferase UGT76G1 mutant library and selection of superior mutants

A gene sequence for a wild-type glycosyltransferase UGT76G1 was subjected to random mutations by error-prone PCR (ep-PCR) to construct a mutant library. With ugt76g1-ATG-F and ugt76g1-TAA-R as primers and a plasmid pRB1k-ugt76g1 as a template, ep-PCR amplification was carried out based on a low-fidelity property of rTaq DNA polymerase (from TAKARA). An amplification system and an amplification program were shown in Tables 1 and 2, respectively.
ugt76gl-ATG-F: ATGGCCGAAAACAAGACCGA (SEQ ID NO: 18).
ugt76g1-TAA-R: TTACAAAGAGGAAATGTAAG (SEQ ID NO: 19).

**Table 1 ep-PCR amplification system**

| Component | Volume | Concentration |
|---|---|---|
| 10×Buffer | 10 µL | 1× |
| 25 mM dNTPs | 4 µL | 1 mM |
| 10 µm primer-F | 5 µL | 0.5 mM |
| 10 µm primer-R | 5 µL | 0.5 mM |
| Template | - | 10 ng |
| 25 mM MgCl₂ | 22 µL | 5.5 mM |
| 5 mM MnCl₂ | 1 µL | 0.05 mM |
| rTaq | 1 µL | |
| ddH₂O | Add to 100 µL | |

**Table 2 ep-PCR amplification program**

| Step | Temperature | Time | |
|---|---|---|---|
| Pre-denaturation | 95°C | 5 min | |
| Denaturation | 95°C | 30 s | 30 cycles |
| Annealing | 58°C | 30 s | |
| Extension | 72°C | 2 kb/min | |
| Additional extension | 72°C | 5 min | |
| | 4°C | Hold | |

PCR products were purified to produce mutated UGT76G1 gene fragments. The mutated UGT76G1 gene fragments were then ligated into a vector pRB1k using a Gibson seamless cloning kit to produce a complete plasmid mutant library including UGT76G1 mutant genes. A Gibson ligation reaction system was shown in Table 3.

**Table 3 Gibson ligation reaction system**

| **Component** | **Volume/µL** |
|---|---|
| Vector DNA | 1-3 |
| Inserted fragment | 1-3 |
| 2× Heiff Clone Enzyme Premix | 5 |
| DDW | To 10 |

A ligation reaction was carried out in a 50°C water bath for 1 h. A Gibson ligation product was transformed into DH5α competent *Escherichia coli* cells. Transformed *Escherichia coli* cells were allowed to recover in a shaker for 1 h, then coated on an agar plate, and cultured in a 37°C incubator for 12 h. After colonies grew, five single colonies were randomly picked and cultured, and a plasmid was then extracted and sequenced. Then, colonies were scraped off an agar plate using a glass rod, and plasmids were extracted to produce a plasmid mutant library, which was stored at -20°C for subsequent high-throughput screening of mutants.

Steps of the high-throughput screening:
(1) After the glycosyltransferase UGT76G1 mutant library was constructed, single colonies were picked from an agar plate using sterile toothpicks (autoclaved), inoculated into a 96-deep-well plate with 800 µL of an LB medium (including kanamycin sulfate) in each well, and cultured in a 96-well plate shaker at 37°C and 900 rpm for 24 h to produce a seed culture.
(2) A small amount of the seed culture was transferred using an inoculation needle to a 96-well plate with an LB medium including kanamycin sulfate (final concentration: 50 µg/mL), and cultured at 37°C and 900 rpm. When OD₆₀₀ of a resulting culture reached 0.6 to 0.8, L-arabinose was added at a final concentration of 1 mM, and induction was conducted in a 96-well plate shaker at 30°C and 900 rpm for 22 h. After the induction was completed, centrifugation was conducted at 3,000 × g for 20 min to collect cells. A lysozyme solution was added to each well, and thorough mixing was conducted to allow sufficient cell lysis, such that intracellular enzymes were released. After the cell lysis was completed, centrifugation was conducted in a refrigerated centrifuge at 4°C and 3,000 × g for 20 min to produce a glycosyltransferase UGT76G1 mutant solution.
(3) Preparation of a sucrose synthase AtSUS solution: A small amount of a seed culture of the recombinant *Escherichia coli* strain expressing the sucrose synthase AtSUS was transferred using an inoculation needle to a conical flask with 10 mL of an LB medium including kanamycin sulfate (final concentration: 50 µg/mL), and cultured in a shaker at 37°C and 220 r/min. When OD₆₀₀ of a resulting culture reached 0.6 to 0.8, L-arabinose was added at a final concentration of 1 mM, and culturing was further conducted at 30°C and 220 r/min for 22 h. Then, centrifugation was conducted to collect cells. The cells were subjected to ultrasonic disruption and then centrifugation at 5,000 × g for 5 min to produce the sucrose synthase AtSUS solution.
(4) 25 mM of STV, 150 mM of sucrose, 2.5 mM of UDPG, 0.16 mL of the sucrose synthase AtSUS solution, 0.16 mL of the glycosyltransferase UGT76G1 mutant solution, and a 100 mM sodium phosphate buffer were mixed to produce a reaction system. The reaction system was incubated in a 37°C water bath for 7 h to allow a reaction, and then heated for 5 min to terminate the reaction. A pH of the reaction system was 7.
(5) 3,5-Dinitrosalicylic acid (DNS) assay: A reaction product solution was centrifuged in a refrigerated centrifuge at 4°C and 3,000 × g for 20 min to produce a first supernatant, and the first supernatant was collected. 70 µL of the first supernatant and 210 µL of DNS were added to a clean 96-well plate, thoroughly mixed, heated for 5 min to allow a DNS color development reaction, cooled to room temperature, and then centrifuged at 3,000 × g for 18 min to produce a second supernatant. 200 µL of the second supernatant was taken and added to a 96-well microplate, and an OD₅₄₀ value was measured by a microplate reader. UGT76G1 mutants leading to reaction product solutions with high OD₅₄₀ values were selected and re-verified in test tubes.
(6) Test tube-scale validation: After the UGT76G1 mutants leading to reaction product solutions with high OD₅₄₀ values were obtained through 96-well plate screening, UGT76G1 mutant-expressing single colonies, wild-type UGT76G1-expressing single colonies, and single colonies of the recombinant *Escherichia coli* strain expressing the sucrose synthase AtSUS were each picked, inoculated into a test tube, and cultured in a shaker at 37°C and 220 rpm for 12 h to produce seed cultures. A seed culture was inoculated at a volume ratio of 1% into 20 mL of an LB medium (including kanamycin sulfate at a final concentration of 50 µg/mL), and cultured under shaking at 37°C and 220 rpm. When OD₆₀₀ of a resulting culture reached 0.6 to 0.8, L-arabinose was added at a final concentration of 1 mM, and induction was conducted at 30°C and 220 rpm for 18 h. After the induction was completed, resulting cells were collected by centrifugation (which was conducted at 5,000 × g for 10 min), resuspended in 0.51 mL of a 100 mM sodium phosphate buffer at a pH of 8.0, and then subjected to disruption and centrifugation to produce glycosyltransferase UGT76G1 mutant solutions, a wild-type glycosyltransferase UGT76G1 solution, and a sucrose synthase AtSUS solution for subsequent experiments.

A test tube-scale reaction system was 10 mL, and included 25 mM of STV, 150 mM of sucrose, 2.5 mM of UDPG, 0.5 mL of a glycosyltransferase UGT76G1 mutant solution or the wild-type glycosyltransferase UGT76G1 solution, 0.2 mL of the sucrose synthase AtSUS solution, and a balance of a 100 mM sodium phosphate buffer. A pH of the test tube-scale reaction system was 7. The test tube-scale reaction system was incubated in a 37°C water bath for 7 h to allow a reaction, then incubated in a boiling water bath for 5 min to terminate the reaction, and centrifuged at 10,000 × g for 2 min to produce a reaction supernatant. The reaction supernatant was collected and subjected to DNS assay. Reaction products resulting from the catalysis of mutants with higher OD₅₄₀ values than the wild-type glycosyltransferase UGT76G1 were tested by high-performance liquid chromatography (HPLC) to verify RA yields.

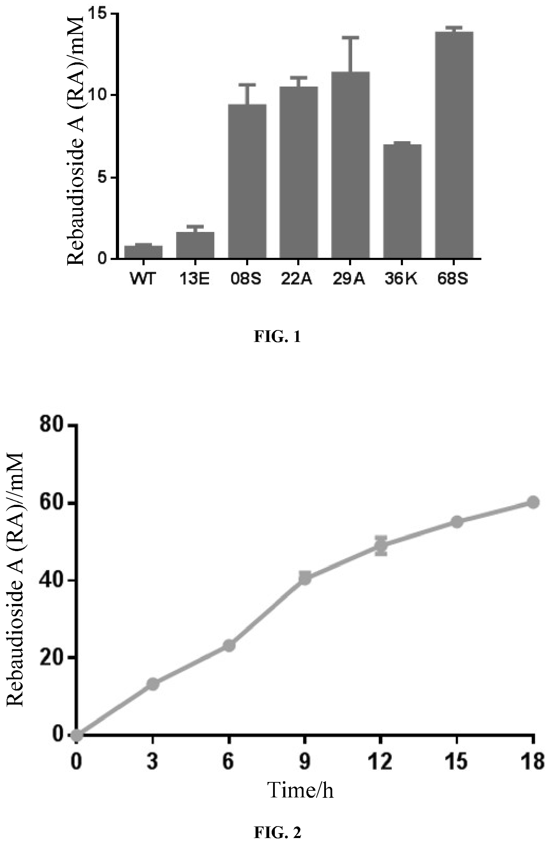

Through the plurality of rounds of primary screening using 96-well plates and the test tube-scale secondary screening, six superior mutants were ultimately selected from nearly 35,000 mutants in the glycosyltransferase UGT76G1 mutant library. These six superior mutants all had a higher enzymatic activity than the wild-type glycosyltransferase, and demonstrated significantly improved efficiency in catalyzing the conversion of STV into RA. Yields of RA corresponding to these superior mutants during the test tube-scale validation were shown in FIG. 1. A yield of RA synthesized under the catalysis of a mutant 68S was more than 30 times higher than a yield of RA synthesized under the catalysis of the wild-type glycosyltransferase UGT76G1. According to sequencing results, the mutant 68S was produced through the following mutations based on an amino acid sequence shown in SEQ ID NO: 1: A 109th amino acid was mutated from L to Q, a 113th amino acid was mutated from S to C, and a 424th amino acid was mutated from I to F.

### Example 4

### Scale-up study on the synthesis of RA with the optimal mutant 68S

Seed cultures of the mutant 68S and the sucrose synthase AtSUS were each inoculated at a volume ratio of 1% into 400 mL of an LB medium (including kanamycin sulfate at a final concentration of 50 µg/mL), and cultured under shaking at 37°C and 220 rpm. When OD₆₀₀ of a resulting culture reached 0.6 to 0.8, L-arabinose was added at a final concentration of 1 mM, and induction was conducted at 30°C and 220 rpm for 18 h. After the induction was completed, resulting cells were collected by centrifugation (which was conducted at 5,000 × g for 10 min), resuspended in 10.5 mL of a 100 mM sodium phosphate buffer at a pH of 8.0, and then subjected to disruption and centrifugation to produce a glycosyltransferase UGT76G1 mutant 68S solution and a sucrose synthase AtSUS solution.

A reaction system was 100 mL, and included 60 mM of STV, 360 mM of sucrose, 3 mM of UDPG, 10 mL of the sucrose synthase AtSUS solution, 10 mL of the glycosyltransferase UGT76G1 mutant 68S solution, and a balance of a 100 mM sodium phosphate buffer. A reaction was conducted at 37°C for 18 h. A pH of the reaction system was 7.

A sample was collected from a reaction solution every 4 h, and tested by HPLC to determine a variation trend of an RA yield over the conversion time. Results were shown in FIG. 2. The optimal mutant 68S could convert 60 mM STV into 60 mM RA within 18 h, that is, STV could be completely converted into RA.

### Example 5

Construction of a recombinant *Escherichia coli* strain AtSUS expressing sucrose synthase AtSUS

With Inf-pYB1k-atsus-F and Inf-pYB1k-atsus-R as primers and cDNA of *Arabidopsis thaliana*-derived sucrose synthase AtSUS as a template, PCR amplification was carried out using a high-fidelity DNA polymerase (purchased from ABclonal Technology Co., Ltd., Wuhan, China) to produce a correct atsus gene fragment. The atsus gene fragment was shown in SEQ ID NO: 6. An amino acid sequence of the sucrose synthase AtSUS was shown in SEQ ID NO: 5.
Inf-pYB1k-atsus-F:
   GCTAACAGGAGGAATTAACCATGGAAAATAAAACGGAGACC (SEQ ID NO: 7).
Inf-pYB1k-atsus-R:
   CCAGATCTACCCTCGAGTTACAACGATGAAATGTAAGAAAC (SEQ ID NO: 8).

The atsus gene fragment was ligated into an expression vector pYB1k by a Gibson assembly method to produce an expression vector pYB1k-atsus.

With pYB1k-F and pYB1k-R as primers and a pYB1k empty vector as a template, PCR amplification was carried out using a high-fidelity DNA polymerase (purchased from ABclonal Technology Co., Ltd., Wuhan, China) to produce a correct pYB1k expression vector fragment (the pYB1k empty vector had been disclosed in You R, Wang L, Shi C, Chen H, Zhang S, Hu M, Tao Y. Efficient production of myo-inositol in Escherichia coli through metabolic engineering. Microb. Cell Fact. 2020 May 24; 19 (1): 109.).
pYB1k-F: CTCGAGGGTAGATCTGGTAC (SEQ ID NO: 9).
pYB1k-R: GGTTAATTCCTCCTGTTAGC (SEQ ID NO: 10).

The atsus gene fragment and the pYB1k expression vector fragment were ligated by a Gibson assembly method.

DH5α competent *Escherichia coli* cells (purchased from TransGen Biotech Co., Ltd., Beijing, China) were prepared by a calcium chloride (CaCl₂) method. A Gibson ligation product was added to the DH5α competent *Escherichia coli* cells. A resulting mixture was incubated on ice for 30 min, then incubated in a 42°C water bath for 90 s, and then placed on ice for 2 min. Transformed *Escherichia coli* cells were added to 1 mL of an LB medium, allowed to recover in a 37°C shaker for 1 h, coated on a kanamycin-containing LB agar plate, and cultured overnight at 37°C.

A plurality of single colonies were picked and cultured. PCR verification was carried out using primers pBAD-F and atsus-F300-R. Positive clones with a target sequence of a correct size were picked and cultured, and a plasmid was then extracted to obtain a positive-clone plasmid pYB1k-atsus.
pBAD-F: GATTATTTGCACGGCGTCAC (SEQ ID NO: 11).
atsus-F300-R: CTTGTGGGTCGTTGTCGAGGATG (SEQ ID NO: 12).

BW25113 competent *Escherichia coli* cells were prepared by a CaCl₂ method. The positive-clone plasmid pYB1k-atsus was transformed into the BW25113 competent *Escherichia coli* cells. Transformed *Escherichia coli* cells were then coated on a kanamycin-containing LB agar plate and cultured overnight at 37°C. Positive clones carrying the plasmid pYB1k-atsus were selected, which was the recombinant *Escherichia coli* strain AtSUS.

### Example 6

Construction of a recombinant *Escherichia coli* strain UGT91C1 expressing a wild-type glycosyltransferase UGT91C1

With ugt91c1-F and ugt91c1-R as primers and cDNA of a wild-type *Oryza* sativa-derived glycosyltransferase UGT91C1 as a template, PCR amplification was carried out using a high-fidelity DNA polymerase (purchased from ABclonal Technology Co., Ltd., Wuhan, China) to produce a correct ugt91c1 gene fragment.
ugt91c1-F: CTTTAAGAAGGAGATATACCATGGATTCGGGTTACTCTTCC (SEQ ID NO: 24).
ugt91c1-R: CTTGTCGACGGAGCTCGAATTCTTAGTCTTTATAGCTACGGAG (SEQ ID NO: 25).

With pET28a-F and pET28a-R as primers and a pET28a empty vector as a template, PCR amplification was carried out using a high-fidelity DNA polymerase (purchased from ABclonal Technology Co., Ltd., Wuhan, China) to produce a correct pET28a expression vector fragment (the pET28a empty vector had been disclosed in Pei Wang, Hai-Yan Zhou, Bo Li, Wen-Qing Ding, Zhi-Qiang Liu, Yu-Guo Zheng, Multiplex modification of Escherichia coli for enhanced β-alanine biosynthesis through metabolic engineering, Bioresource Technology, Volume 342, 2021, 126050.).
pET28a-F: GAATTCGAGCTCCGTCGACAAG (SEQ ID NO: 26).
pET28a-R: GGTATATCTC CTTCTTAAAG (SEQ ID NO: 27).

The ugt91c1 gene fragment and the pET28a expression vector fragment were ligated by a Gibson assembly method.

DH5α competent *Escherichia coli* cells (purchased from TransGen Biotech Co., Ltd., Beijing, China) were prepared by a CaCl₂ method. A Gibson ligation product was added to the DH5α competent *Escherichia coli* cells. A resulting mixture was incubated on ice for 30 min, then incubated in a 42°C water bath for 90 s, and then placed on ice for 2 min. Transformed *Escherichia coli* cells were added to 1 mL of an LB medium, allowed to recover in a 37°C shaker for 1 h, coated on a kanamycin-containing LB agar plate, and cultured overnight at 37°C.

A plurality of single colonies were picked and cultured. PCR verification was carried out using primers T7-F and ugt91c1-F300-R. Positive clones with a target sequence of a correct size were picked and cultured, and a plasmid was then extracted to obtain a positive-clone plasmid pET28a-ugt91cl.
T7-F: TAATACGACTCACTATAGGG (SEQ ID NO: 28).
ugt91c1-F300-R: GGCGGTGCAG TTCAACCATG (SEQ ID NO: 29).

BL21 (DE3) competent *Escherichia coli* cells were prepared by a CaCl₂ method. The positive-clone plasmid pET28a-ugt91c1 was transformed into the BL21 (DE3) competent *Escherichia coli* cells. Transformed *Escherichia coli* cells were then coated on a kanamycin-containing LB agar plate and cultured overnight at 37°C. Positive clones carrying the plasmid pET28a-ugt91c1 were selected, which was the recombinant *Escherichia coli* strain UGT91C1.

### Example 7

### Construction of a glycosyltransferase UGT91C1 mutant library

UGT91C1 was subjected to random mutations by ep-PCR. With ugt91c1-ATG-F and ugt91c1-TAA-R as primers and a plasmid pET28a-ugt91c1 as a template, ep-PCR amplification was carried out based on a low-fidelity property of rTaq DNA polymerase (from TAKARA). An amplification system and an amplification program were shown in Tables 4 and 5, respectively.
ugt91c1-ATG-F: ATGGCCGAAAACAAGACCGA (SEQ ID NO: 18).
ugt91c1-TAA-R: TTACAAAGAGGAAATGTAAG (SEQ ID NO: 19).

**Table 4 ep-PCR amplification system**

| Component | Volume | Concentration |
|---|---|---|
| 10×Buffer | 10 µL | 1× |
| 25 mM dNTPs | 4 µL | 1 mM |
| 10 µm primer-F | 5 µL | 0.5 mM |
| 10 µm primer-R | 5 µL | 0.5 mM |
| Template | - | 10 ng |
| 25 mM MgCl₂ | 22 µL | 5.5 mM |
| 5 mM MnCl₂ | 1 µL | 0.05 mM |
| rTaq | 1 µL | |
| ddH₂O | Add to 100 µL | |

**Table 5 ep-PCR amplification program**

| Step | Temperature | Time | |
|---|---|---|---|
| Pre-denaturation | 95°C | 5 min | |
| Denaturation | 95°C | 30 s | |
| Annealing | 58°C | 30 s | 30 cycles |
| Extension | 72°C | 2 kb/min | |
| Additional extension | 72°C | 5 min | |
| | 16°C | Hold | |

PCR products were purified to produce mutated UGT91C1 gene fragments. The mutated UGT91C1 gene fragments were then ligated into a vector pET28a using a Gibson seamless cloning kit to produce a complete plasmid mutant library including UGT91C1 mutant genes. A Gibson ligation reaction system was shown in Table 6.

**Table 6 Gibson ligation reaction system**

| **Component** | **Volume/µL** |
|---|---|
| Vector DNA | 1-3 |
| Inserted fragment | 1-3 |
| 2× Heiff Clone Enzyme Premix | 5 |
| DDW | To 10 |

A ligation reaction was carried out in a 50°C water bath for 1 h. A resulting plasmid carrying a UGT91C1 mutant gene was transformed into DH5α competent *Escherichia coli* cells. Transformed *Escherichia coli* cells were allowed to recover in a shaker for 1 h, then coated on an agar plate, and cultured in a 37°C incubator for 12 h. After colonies grew, five single colonies were randomly picked and cultured, and a plasmid was then extracted and sequenced. Then, colonies were scraped off an agar plate using a glass rod, and plasmids were extracted to produce a plasmid mutant library, which was stored at -20°C for subsequent high-throughput screening of mutants.

### Example 8

### High-throughput screening of mutants to obtain superior mutants

After the mutant library was constructed, single colonies were picked from an agar plate using sterile toothpicks (autoclaved), inoculated into a 96-deep-well plate with 800 µL of an LB medium (including kanamycin sulfate) in each well, and cultured in a 96-well plate shaker at 37°C and 900 rpm for 24 h to produce a seed culture.

A small amount of the seed culture was transferred using an inoculation needle to a 96-well plate with an LB medium including kanamycin sulfate (final concentration: 50 µg/mL), and cultured at 37°C and 900 rpm. When OD₆₀₀ of a resulting culture reached 0.6 to 0.8, IPTG was added at a final concentration of 0.4 mM, and induction was conducted in a 96-well plate shaker at 30°C and 900 rpm for 22 h. After the induction was completed, centrifugation was conducted at 3,000 × g for 20 min to collect cells. A lysozyme solution was added to each well, and thorough mixing was conducted to allow sufficient cell lysis, such that intracellular enzymes were released. After the cell lysis was completed, centrifugation was conducted in a refrigerated centrifuge at 4°C and 3,000 × g for 20 min to produce a glycosyltransferase UGT91C1 mutant solution.

Preparation of a sucrose synthase AtSUS solution: A small amount of a seed culture of the recombinant *Escherichia coli* strain AtSUS was transferred using an inoculation needle to a conical flask with 10 mL of an LB medium including kanamycin sulfate (final concentration: 50 µg/mL), and cultured in a shaker at 37°C and 220 r/min. When OD₆₀₀ of a resulting culture reached 0.6 to 0.8, L-arabinose was added at a final concentration of 1 mM, and culturing was further conducted at 30°C and 220 r/min for 22 h. Then, centrifugation was conducted to collect cells. The cells were subjected to ultrasonic disruption and then centrifugation at 5,000 × g for 5 min to produce the sucrose synthase AtSUS solution.

A reaction system was produced by mixing 7 mM of RA, 42 mM of sucrose, 1.6 mM of UDPG, 0.16 mL of the sucrose synthase AtSUS solution, 0.16 mL of the glycosyltransferase UGT91C1 mutant solution or the wild-type glycosyltransferase UGT91C1 solution, and a 100 mM sodium phosphate buffer. The reaction system was incubated in a 37°C water bath to allow a reaction for 6 h and then heated for 5 min to terminate the reaction. A pH of the reaction system was 8.

DNS assay: A reaction product solution was centrifuged in a refrigerated centrifuge at 4°C and 3,000 × g for 20 min to produce a first supernatant, and the first supernatant was collected. 70 µL of the first supernatant and 210 µL of DNS were added to a clean 96-well plate, thoroughly mixed, heated for 5 min to allow a DNS color development reaction, cooled to room temperature, and then centrifuged at 3,000 × g for 18 min to produce a second supernatant. 200 µL of the second supernatant was taken and added to a 96-well microplate, and an OD₅₄₀ value was measured by a microplate reader. UGT91C1 mutants leading to reaction product solutions with high OD₅₄₀ values were identified as mutants with high activity, and were subsequently re-verified in test tubes.

Test tube-scale validation: After the UGT91C1 mutants corresponding to high OD₅₄₀ values were obtained through 96-well plate screening, UGT91C1 mutant-expressing single colonies, wild-type UGT91C1-expressing single colonies, and single colonies of the recombinant *Escherichia coli* strain AtSUS were each picked, inoculated into a test tube, and cultured in a shaker at 37°C and 220 rpm for 12 h to produce seed cultures. A seed culture was inoculated at a volume ratio of 1% into 20 mL of an LB medium (including kanamycin sulfate at a final concentration of 50 µg/mL), and cultured under shaking at 37°C and 220 rpm. When OD₆₀₀ of a resulting culture reached 0.6 to 0.8, IPTG was added at a final concentration of 0.4 mM to each of media including the UGT91C1 mutant-expressing single colonies and a medium including the wild-type UGT91C1-expressing single colonies, and L-arabinose was added at a final concentration of 1 mM to a medium including single colonies of the recombinant *Escherichia coli* strain AtSUS. Then, induction was conducted at 30°C and 220 rpm for 15 h. After the induction was completed, resulting cells were collected by centrifugation (which was conducted at 5,000 × g for 10 min), resuspended in 0.51 mL of a 100 mM sodium phosphate buffer at a pH of 8.0, and then subjected to disruption and centrifugation to produce glycosyltransferase UGT91C1 mutant solutions, a wild-type glycosyltransferase UGT91C1 solution, and a sucrose synthase AtSUS solution.

A test tube-scale reaction system was 10 mL, and included 8 mM of RA, 48 mM of sucrose, 1.6 mM of UDPG, 0.5 mL of a glycosyltransferase UGT91C1 mutant solution or the wild-type glycosyltransferase UGT91C1 solution, 0.2 mL of the sucrose synthase AtSUS solution, and a balance of a 100 mM sodium phosphate buffer. A pH of the test tube-scale reaction system was 8. The test tube-scale reaction system was incubated in a 37°C water bath for 6 h to allow a reaction, then incubated in a boiling water bath for 5 min to terminate the reaction, and centrifuged at 10,000 × g for 2 min to produce a reaction supernatant. The reaction supernatant was collected and subjected to DNS assay. Reaction products resulting from the catalysis of mutants with higher OD₅₄₀ values than the wild-type glycosyltransferase UGT91C1 were tested by HPLC to verify RD yields.

### Screening results are as follows:

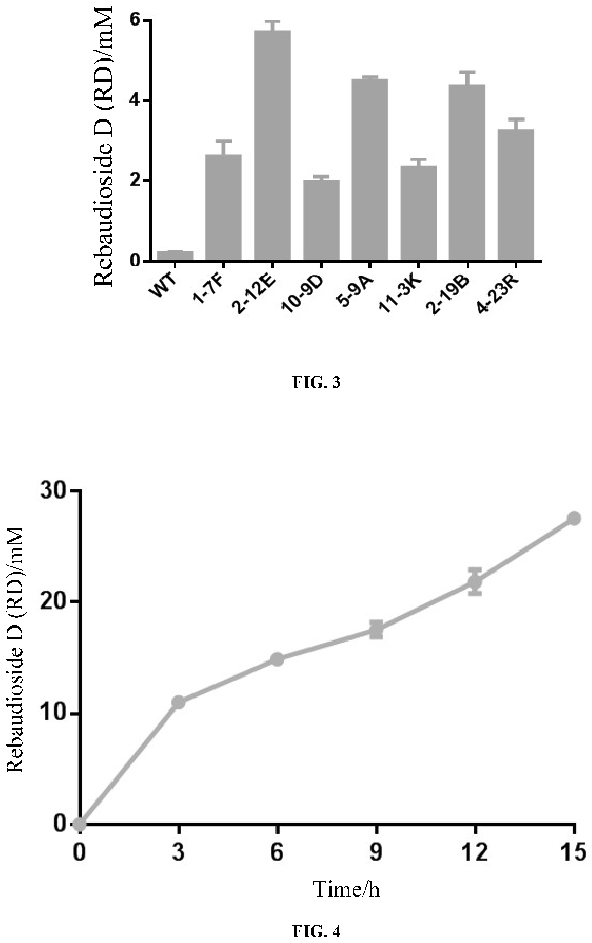

Through the plurality of rounds of screening, seven superior mutants were ultimately selected by the inventors from nearly 500,000 mutants in the glycosyltransferase UGT91C1 mutant library. These seven superior mutants all had a higher enzymatic activity than the wild-type glycosyltransferase, and demonstrated significantly improved efficiency in catalyzing the conversion of RA into RD. Test results of RD yields were shown in FIG. 3. A yield of RD synthesized under the catalysis of the optimal mutant 2-12E was nearly 30 times higher than a yield of RD synthesized under the catalysis of the wild-type glycosyltransferase UGT91C1. According to sequencing results, an amino acid sequence of the mutant 2-12E was produced through the following mutations based on an amino acid sequence shown in SEQ ID NO: 20: A 89th amino acid was mutated from N to Y, a 155th amino acid was mutated from M to L, a 274th amino acid was mutated from S to T, and a 361st amino acid was mutated from N to S.

### Example 9

### Scale-up study on the synthesis of RD with the optimal mutant 2-12E

Seed cultures of the mutant 2-12E and the sucrose synthase AtSUS were each inoculated at a volume ratio of 1% into 400 mL of an LB medium (including kanamycin sulfate at a final concentration of 50 µg/mL), and cultured under shaking at 37°C and 220 rpm. When OD₆₀₀ of a resulting culture reached 0.6 to 0.8, IPTG was added at a final concentration of 0.4 mM and L-arabinose was added at a final concentration of 1 mM, respectively. Then, induction was conducted at 30°C and 220 rpm for 15 h. After the induction was completed, resulting cells were collected by centrifugation (which was conducted at 5,000 × g for 10 min), resuspended in 10.5 mL of a 100 mM sodium phosphate buffer at a pH of 8.0, and then subjected to disruption and centrifugation to produce a glycosyltransferase UGT91C1 mutant 68S solution and a sucrose synthase AtSUS solution.

A reaction system was 100 mL, and included 27.4 mM of RA, 164.4 mM of sucrose, 1 mM of UDPG, 10 mL of the sucrose synthase AtSUS solution, 10 mL of the glycosyltransferase UGT91C1 mutant 2-12E solution, and a balance of a 100 mM sodium phosphate buffer. Reaction conditions: A pH of the reaction system was 8. A reaction was conducted at 37°C for 15 h.

A sample was collected from a reaction solution every 4 h, and tested by HPLC to determine a variation trend of an RD yield over the conversion time. Results were shown in FIG. 4. The optimal mutant 2-12E could convert 27.4 mM RA into 25.8 mM RD within 15 h, with a conversion rate of 94.2% for RA.

### Example 10

### Construction of a recombinant Escherichia coli engineered strain AtSUS

With Inf-pYB1k-atsus-F and Inf-pYB1k-atsus-R as primers and cDNA of a gene atsus of *Arabidopsis thaliana*-derived sucrose synthase as a template, PCR amplification was carried out using a high-fidelity DNA polymerase (purchased from ABclonal Technology Co., Ltd., Wuhan, China) to produce a correct atsus gene fragment. The atsus gene fragment was shown in SEQ ID NO: 6. An amino acid sequence of the sucrose synthase AtSUS was shown in SEQ ID NO: 5.
Inf-pYB1k-atsus-F:
   GCTAACAGGAGGAATTAACCATGGAAAATAAAACGGAGACC (SEQ ID NO: 7).
Inf-pYB1k-atsus-R:
   CCAGATCTACCCTCGAGTTACAACGATGAAATGTAAGAAAC (SEQ ID NO: 8).

With pYB1k-F and pYB1k-R as primers and a pYB1k empty vector as a template, PCR amplification was carried out using a high-fidelity DNA polymerase (purchased from ABclonal Technology Co., Ltd., Wuhan, China) to produce a correct pYB1k expression vector fragment (the pYB1k empty vector had been disclosed in You R, Wang L, Shi C, Chen H, Zhang S, Hu M, Tao Y. Efficient production of myo-inositol in Escherichia coli through metabolic engineering. Microb. Cell Fact. 2020 May 24; 19 (1): 109.).
pYB1k-F: CTCGAGGGTAGATCTGGTAC (SEQ ID NO: 9).
pYB1k-R: GGTTAATTCCTCCTGTTAGC (SEQ ID NO: 10).

The atsus gene fragment was ligated into an expression vector pYB1k by a Gibson assembly method to produce an expression vector pYB1k-atsus.

DH5α competent *Escherichia coli* cells (purchased from TransGen Biotech Co., Ltd., Beijing, China) were prepared by a CaCl₂ method. A Gibson ligation product was added to the DH5α competent *Escherichia coli* cells. A resulting mixture was incubated on ice for 30 min, then incubated in a 42°C water bath for 90 s, and then placed on ice for 2 min. Transformed *Escherichia coli* cells were added to 1 mL of an LB medium, allowed to recover in a 37°C shaker for 1 h, coated on a kanamycin-containing LB agar plate, and cultured overnight at 37°C.

A plurality of single colonies were picked and cultured. PCR verification was carried out using primers pBAD-F and atsus-F300-R. Positive clones with a target sequence of a correct size were picked and cultured, and a plasmid was then extracted to obtain a positive-clone plasmid pYB1k-atsus.
pBAD-F: GATTATTTGCACGGCGTCAC (SEQ ID NO: 11).
atsus-F300-R: CTTGTGGGTCGTTGTCGAGGATG (SEQ ID NO: 12).

BW25113 competent *Escherichia coli* cells were prepared by a CaCl₂ method. The plasmid pYB1k-atsus1 was transformed into the BW25113 competent *Escherichia coli* cells. Transformed *Escherichia coli* cells were then coated on a kanamycin-containing LB agar plate and cultured overnight at 37°C. Positive clones carrying the plasmid pYB1k-atsus1 were selected, which was the recombinant *Escherichia coli* engineered strain AtSUS.

### Example 11

Construction of a recombinant *Escherichia coli* engineered strain expressing a wild-type glycosyltransferase UGT76G1

Based on a nucleotide sequence for a wild-type *Stevia rebaudiana*-derived glycosyltransferase UGT76G1 provided by the National Center for Biotechnology Information (NCBI) database, a full-length sequence was synthesized by General Biosystems (Anhui) Co., Ltd. The nucleotide sequence for the wild-type *Stevia rebaudiana*-derived glycosyltransferase UGT76G1 was then cloned into a vector pET28a to produce a recombinant expression vector pET28a-ugt76g1. (the vector pET28a had been disclosed in Pei Wang, Hai-Yan Zhou, Bo Li, Wen-Qing Ding, Zhi-Qiang Liu, Yu-Guo Zheng, Multiplex modification of Escherichia coli for enhanced β-alanine biosynthesis through metabolic engineering, Bioresource Technology, Volume 342, 2021, 126050.).

With Inf-pYB1k-ugt76g1-F and Inf-pYB1k-ugt76g1-R as primers and pET28a-ugt76g1 as a template, PCR amplification was carried out using a high-fidelity DNA polymerase (purchased from ABclonal Technology Co., Ltd., Wuhan, China) to produce a correct ugt76g1 gene fragment.
Inf-pYB1k-ugt76g1-F:
   CTAACAGGAGGAATTAACCATATGGCCGAAAACAAGACCGA (SEQ ID NO: 13).
Inf-pYB1k-ugt76g1-R:
   GTACCAGATCTACCCTCGAGTTACAAAGAGGAAATGTAAG (SEQ ID NO: 32).

With pYB1k-F and pYB1k-R as primers and a pYB1k empty vector as a template, PCR amplification was carried out using a high-fidelity DNA polymerase (purchased from ABclonal Technology Co., Ltd., Wuhan, China) to produce a correct pYB1k expression vector fragment
pYB1k-F: CTCGAGGGTAGATCTGGTAC (SEQ ID NO: 15).
pYB1k-R: GGTTAATTCCTCCTGTTAGC (SEQ ID NO: 16).

The ugt76g1 gene fragment was ligated into an empty vector pYB1k by a Gibson assembly method to produce an expression vector pYB1k-ugt76g1.

DH5α competent *Escherichia coli* cells (purchased from TransGen Biotech Co., Ltd., Beijing, China) were prepared by a CaCl₂ method. A Gibson ligation product was added to the DH5α competent *Escherichia coli* cells. A resulting mixture was incubated on ice for 30 min, then incubated in a 42°C water bath for 90 s, and then placed on ice for 2 min. Transformed *Escherichia coli* cells were added to 1 mL of an LB medium, allowed to recover in a 37°C shaker for 1 h, coated on a kanamycin-containing LB agar plate, and cultured overnight at 37°C.

A plurality of single colonies were picked and cultured. PCR verification was carried out using primers pBAD-F and ugt76g1-F300-R. Positive clones with a target sequence of a correct size were picked and cultured, and a plasmid was then extracted to obtain a positive-clone plasmid pYB1k-ugt76g1.
pBAD-F: GATTATTTGCACGGCGTCAC (SEQ ID NO: 11).
ugt76g1-F300-R: CTGCAGTTCCAGTTCACGAC (SEQ ID NO: 17).

BW25113 competent *Escherichia coli* cells were prepared by a CaCl₂ method. The plasmid pYB1k-ugt76g1 was transformed into the BW25113 competent *Escherichia coli* cells. Transformed *Escherichia coli* cells were then coated on a kanamycin-containing LB agar plate and cultured overnight at 37°C. Positive clones carrying the plasmid pYB1k-ugt76g1 were selected, which were the recombinant *Escherichia coli* engineered strain expressing the wild-type glycosyltransferase UGT76G1.

### Example 12

### Construction of a glycosyltransferase UGT76G1 mutant library

A gene sequence for a wild-type glycosyltransferase UGT76G1 was subjected to random mutations by ep-PCR to construct a mutant library. With ugt76g1-ATG-F and ugt76g1-TAA-R as primers and a plasmid pYB1k-ugt76g1 as a template, ep-PCR amplification was carried out based on a low-fidelity property of rTaq DNA polymerase (from TAKARA). An amplification system and an amplification program were shown in Tables 7 and 8, respectively.
ugt76g1-ATG-F: ATGGCCGAAAACAAGACCGA (SEQ ID NO: 18).
ugt76g1-TAA-R: TTACAAAGAGGAAATGTAAG (SEQ ID NO: 19).

**Table 7 ep-PCR amplification system**

| Component | Volume | Concentration |
|---|---|---|
| 10×Buffer | 10 µL | 1× |
| 25 mM dNTPs | 4 µL | 1 mM |
| 10 µm primer-F | 5 µL | 0.5 mM |
| 10 µm primer-R | 5 µL | 0.5 mM |
| Template | - | 10 ng |
| 25 mM MgCl₂ | 22 µL | 5.5 mM |
| 5 mM MnCl₂ | 1 µL | 0.05 mM |
| rTaq | 1 µL | |
| ddH₂O | Add to 100 µL | |

**Table 8 ep-PCR amplification program**

| Step | Temperature | Time | |
|---|---|---|---|
| Pre-denaturation | 95°C | 5 min | |
| Denaturation | 95°C | 30 s | |
| Annealing | 58°C | 30 s | 30 cycles |
| Extension | 72°C | 2 kb/min | |
| Additional extension | 72°C | 5 min | |
| | 16°C | Hold | |

PCR products were purified to produce mutated UGT76G1 gene fragments. The mutated UGT76G1 gene fragments were then ligated into a vector pYB1k using a Gibson seamless cloning kit to produce a complete plasmid mutant library including UGT76G1 mutant genes. A Gibson ligation reaction system was shown in Table 9.

**Table 9 Gibson ligation reaction system**

| **Component** | **Volume/µL** |
|---|---|
| Vector DNA | 1-3 |
| Inserted fragment | 1-3 |
| 2× Heiff Clone Enzyme Premix | 5 |
| DDW | To 10 |

A ligation reaction was carried out in a 50°C water bath for 1 h. A resulting plasmid carrying a UGT76G1 mutant gene was transformed into DH5α competent *Escherichia coli* cells. Transformed *Escherichia coli* cells were allowed to recover in a shaker for 1 h, then coated on an agar plate, and cultured in a 37°C incubator for 12 h. After colonies grew, five single colonies were randomly picked and cultured, and a plasmid was then extracted and sequenced. Then, colonies were scraped off an agar plate using a glass rod, and plasmids were extracted to produce a plasmid library, which was stored at -20°C for subsequent high-throughput screening of mutants.

### Example 13

### High-throughput screening

After the mutant library was constructed, single colonies were picked from an agar plate using sterile toothpicks (autoclaved), inoculated into a 96-deep-well plate with 800 µL of an LB medium (including kanamycin sulfate) in each well, and cultured in a 96-well plate shaker at 37°C and 900 rpm for 24 h to produce a seed culture.

A small amount of the seed culture was transferred using an inoculation needle to a 96-well plate with an LB medium including kanamycin sulfate (final concentration: 50 µg/mL), and cultured at 37°C and 900 rpm. When OD₆₀₀ of a resulting culture reached 0.6 to 0.8, L-arabinose was added at a final concentration of 1 mM, and induction was conducted in a 96-well plate shaker at 30°C and 900 rpm for 22 h. After the induction was completed, centrifugation was conducted at 3,000 × g for 20 min to collect cells. A lysozyme solution was added to each well, and thorough mixing was conducted to allow sufficient cell lysis, such that intracellular enzymes were released. After the cell lysis was completed, centrifugation was conducted in a refrigerated centrifuge at 4°C and 3,000 × g for 20 min to produce a glycosyltransferase UGT76G1 mutant solution for a subsequent reaction.

Preparation of a sucrose synthase AtSUS solution: A small amount of a seed culture of the recombinant *Escherichia coli* engineered strain AtSUS was transferred using an inoculation needle to a conical flask with 10 mL of an LB medium including kanamycin sulfate (final concentration: 50 µg/mL), and cultured in a shaker at 37°C and 220 r/min. When OD₆₀₀ of a resulting culture reached 0.6 to 0.8, L-arabinose was added at a final concentration of 1 mM, and culturing was further conducted at 30°C and 220 r/min for 22 h. Then, centrifugation was conducted to collect cells. The cells were subjected to ultrasonic disruption and then centrifugation at 5,000 × g for 5 min to produce the sucrose synthase AtSUS solution.

A reaction system was produced by mixing 8 mM of RD, 48 mM of sucrose, 1.6 mM of UDPG, 0.16 mL of the sucrose synthase AtSUS solution, 0.16 mL of the glycosyltransferase UGT76G1 mutant solution, and a 100 mM sodium phosphate buffer. A pH of the reaction system was 8. The reaction system was incubated in a 37°C water bath for 6 h to allow a reaction, and then heated for 5 min to terminate the reaction.

DNS assay: A reaction product solution was centrifuged in a refrigerated centrifuge at 4°C and 3,000 × g for 20 min to produce a first supernatant, and the first supernatant was collected. 70 µL of the first supernatant and 210 µL of DNS were added to a clean 96-well plate, thoroughly mixed, heated for 5 min to allow a DNS color development reaction, cooled to room temperature, and then centrifuged at 3,000 × g for 18 min to produce a second supernatant. 200 µL of the second supernatant was taken and added to a 96-well microplate, and an OD₅₄₀ value was measured by a microplate reader. UGT76G1 mutants leading to reaction product solutions with high OD₅₄₀ values were identified as mutants with high activity, and were subsequently re-verified in test tubes.

Test tube-scale validation: After the UGT76G1 mutants corresponding to high OD₅₄₀ values were obtained through 96-well plate screening, glycosyltransferase UGT76G1 mutant-expressing single colonies, wild-type glycosyltransferase UGT76G1-expressing single colonies, and single colonies of the recombinant *Escherichia coli* engineered strain AtSUS were each picked, inoculated into a test tube, and cultured in a shaker at 37°C and 220 rpm for 12 h to produce seed cultures. A seed culture was inoculated at a volume ratio of 1% into 20 mL of an LB medium (including kanamycin sulfate at a final concentration of 50 µg/mL), and cultured under shaking at 37°C and 220 rpm. When OD₆₀₀ of a resulting culture reached 0.6 to 0.8, L-arabinose was added at a final concentration of 1 mM, and induction was conducted at 30°C and 200 rpm for 18 h. After the induction was completed, resulting cells were collected by centrifugation (which was conducted at 5,000 × g for 10 min), resuspended in 0.51 mL of a 100 mM sodium phosphate buffer at a pH of 8.0, and then subjected to disruption and centrifugation to produce glycosyltransferase UGT76G1 mutant solutions, a wild-type glycosyltransferase UGT76G1 solution, and a sucrose synthase AtSUS solution.

A reaction system was 10 mL, and included 8 mM of RD, 48 mM of sucrose, 1.6 mM of UDPG, 0.5 mL of a glycosyltransferase UGT76G1 mutant solution or the wild-type glycosyltransferase UGT76G1 solution, 0.2 mL of the sucrose synthase AtSUS solution, and a balance of a 100 mM sodium phosphate buffer. A pH of the reaction system was 8. The reaction system was incubated in a 37°C water bath for 6 h to allow a reaction, then incubated in a boiling water bath for 5 min to terminate the reaction, and centrifuged at 10,000 × g for 2 min to produce a reaction supernatant. The reaction supernatant was collected and subjected to DNS assay. Reaction products resulting from the catalysis of mutants with higher OD₅₄₀ values than the wild-type glycosyltransferase UGT76G1 were tested by HPLC to verify RM yields.

### Screening results:

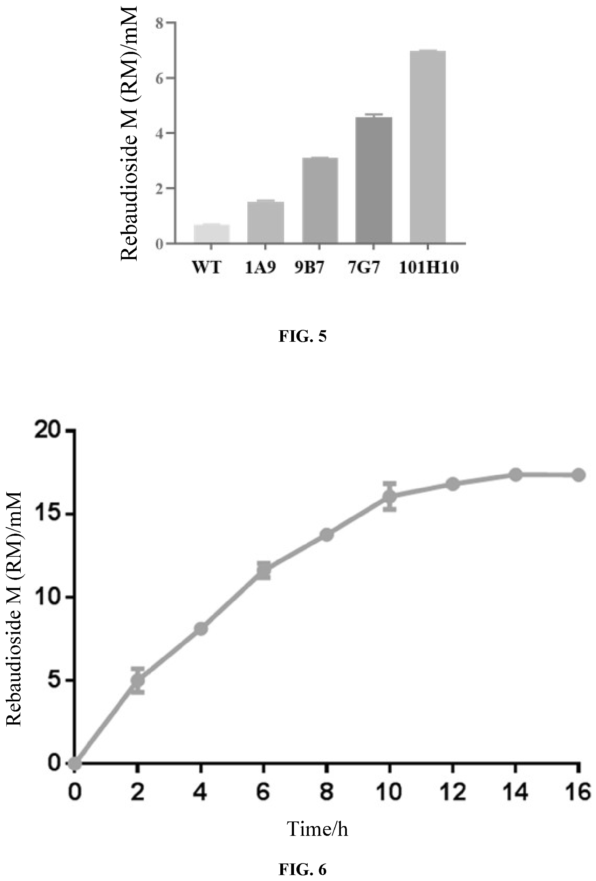

Through the plurality of rounds of primary screening and the test tube-scale secondary screening, four superior mutants were ultimately selected by the inventors from nearly 2,000 mutants in the glycosyltransferase UGT76G1 mutant library. These four superior mutants all had a higher enzymatic activity than the wild-type glycosyltransferase, and demonstrated significantly improved efficiency in catalyzing the conversion of RD into RM. Yields of RM corresponding to these superior mutants during the test tube-scale validation were shown in FIG. 5. A yield of RM synthesized under the catalysis of a mutant 101H10 was nearly 10 times higher than a yield of RM synthesized under the catalysis of the wild-type glycosyltransferase UGT76G1. According to sequencing results, an amino acid sequence of the mutant 2-12E was produced through the following mutations based on an amino acid sequence shown in SEQ ID NO: 1: An 89th amino acid was mutated from M to H, a 380th amino acid was mutated from L to M, and a 411th amino acid was mutated from A to Y.

### Example 14

### Scale-up study on the synthesis of RM with the optimal mutant 101H10

Seed cultures of the mutant 101H10 and the sucrose synthase AtSUS were each inoculated at a volume ratio of 1% into 400 mL of an LB medium (including kanamycin sulfate at a final concentration of 50 µg/mL), and cultured under shaking at 37°C and 220 rpm. When OD₆₀₀ of a resulting culture reached 0.6 to 0.8, L-arabinose was added at a final concentration of 1 mM, and induction was conducted at 30°C and 220 rpm for 18 h. After the induction was completed, resulting cells were collected by centrifugation (which was conducted at 5,000 × g for 10 min), resuspended in 10.5 mL of a 100 mM sodium phosphate buffer at a pH of 8.0, and then subjected to disruption and centrifugation to produce a glycosyltransferase UGT76G1 mutant 68S solution and a sucrose synthase AtSUS solution.

A reaction system was 100 mL, and included 20 mM of RD, 120 mM of sucrose, 1 mM of UDPG, 10 mL of the sucrose synthase AtSUS1 solution, 10 mL of the glycosyltransferase mutant 101H10 solution, and a balance of a sodium phosphate buffer. A pH of the reaction system was 8. Reaction conditions: A reaction was conducted at 37°C for 16 h.

A sample was collected from a reaction solution every 1 h, and tested by HPLC to determine a variation trend of an RM yield over the conversion time. Results were shown in FIG. 6. The optimal mutant 101H10 could convert 20 mM RD into 17.2 mM RM within 16 h, with a conversion rate of 86% for RD.

The above are only preferred examples of the present disclosure and are not intended to limit the present disclosure. Although the present disclosure is described in detail with reference to the aforementioned examples, those skilled in the art can still make modifications to the technical solutions described in the aforementioned examples, or make equivalent replacement to some technical features. Any modifications, equivalent substitutions, improvements, etc. made within the spirit and principle of the present disclosure should be included within the protection scope of the present disclosure.

## Claims

1. A glycosyltransferase UGT76G1 mutant, wherein the glycosyltransferase UGT76G1 mutant is any one selected from the group consisting of following (A) to (C):
(A) a protein produced through any one or more selected from the group consisting of following mutations based on an amino acid sequence shown in SEQ ID NO: 1:
a mutation of a 109th amino acid from L to Q;
a mutation of a 113th amino acid from S to C; and
a mutation of a 424th amino acid from I to F;
(B) a protein that has an identity of 95% or more with and a same function as an amino acid sequence defined in the (A); and
(C) a fusion protein produced by linking a tag to a terminus of the protein defined in the (A) or the (B).

2. The glycosyltransferase UGT76G1 mutant according to claim 1, wherein an amino acid sequence of the glycosyltransferase UGT76G1 mutant is shown in SEQ ID NO: 3.

3. A biological material selected from the group consisting of following:
(A) a gene encoding the glycosyltransferase UGT76G1 mutant according to claim 1 or 2;
(B) a recombinant plasmid carrying the gene in the (A); and
(C) a recombinant cell carrying the recombinant plasmid or the gene encoding the glycosyltransferase UGT76G1 mutant.

4. An enzyme composition, comprising: a glycosyltransferase UGT76G1 mutant and sucrose synthase AtSUS, wherein the glycosyltransferase UGT76G1 mutant is the glycosyltransferase UGT76G1 mutant according to claim 1 or 2; and
the sucrose synthase AtSUS is following (B1) or (B2): (B1) an amino acid sequence shown in SEQ ID NO: 5; and
(B2) a protein that has an identity of 95% or more with and a same function as the amino acid sequence defined in the (B1).

5. A complete set of recombinant strains expressing the enzyme composition according to claim 4, comprising a recombinant strain A and a recombinant strain B, wherein the recombinant strain A comprises a recombinant plasmid A, and the recombinant plasmid A is produced by constructing a gene encoding the glycosyltransferase UGT76G1 mutant according to claim 1 or 2 into an expression vector; and the recombinant strain B comprises a recombinant plasmid B, and the recombinant plasmid B is produced by constructing a gene encoding the sucrose synthase AtSUS into a plasmid.

6. The complete set of recombinant strains according to claim 5, wherein host strains comprise, but are not limited to, *Escherichia coli*, *Saccharomyces cerevisiae*, *Pichia pastoris*, or *Corynebacterium glutamicum*.

7. A method for synthesizing a rebaudioside A (RA) under catalysis of a glycosyltransferase UGT76G1 mutant, comprising following steps:
adding stevioside (STV), uridine diphosphate glucose (UDPG), sucrose, and the enzyme composition according to claim 4 or inducibly expressed enzyme products of the complete set of recombinant strains according to claim 5 to a catalytic reaction system, conducting a reaction, and conducting enzyme inactivation and centrifugation to produce a supernatant comprising the RA.

8. The method according to claim 7, wherein the inducibly expressed enzyme products of the complete set of recombinant strains according to claim 5 comprise an inducibly expressed enzyme product A and an inducibly expressed enzyme product B;
a process for acquiring the inducibly expressed enzyme product A comprises following steps:
inoculating a seed culture of the recombinant strain A according to claim 5 into a medium comprising kanamycin sulfate, and culturing; when OD₆₀₀ of a resulting culture reaches 0.6 to 0.8, adding L-arabinose, and conducting induction for 8 h to 40 h; conducting centrifugation to produce a first cell pellet, and collecting the first cell pellet; subjecting the first cell pellet to disruption, conducting centrifugation to produce a first supernatant, and collecting the first supernatant, which is the inducibly expressed enzyme product A, wherein an inoculum size is 1 v/v%, a final concentration of the kanamycin sulfate is 10 µg/mL to 100 µg/mL, and a final concentration of the L-arabinose is 0.1 mM to 15 mM; and
a process for acquiring the inducibly expressed enzyme product B comprises following steps:
inoculating a seed culture of the recombinant strain B according to claim 5 into a medium comprising kanamycin sulfate, and culturing at 30°C to 40°C and 200 r/min to 300 r/min until OD₆₀₀ of a resulting culture reaches 0.6 to 0.8; adding L-arabinose, and further culturing for 8 h to 40 h; conducting centrifugation to produce a second cell pellet, and collecting the second cell pellet; subjecting the second cell pellet to disruption, conducting centrifugation to produce a second supernatant, and collecting the second supernatant, which is the inducibly expressed enzyme product B, wherein an inoculum size is 1 v/v%, a final concentration of the kanamycin sulfate is 10 µg/mL to 100 µg/mL, and a final concentration of the L-arabinose is 0.1 mM to 15 mM.

9. The method according to claim 7 or 8, wherein in the catalytic reaction system, a concentration of the STV is 10 mM to 100 mM, a concentration of the UDPG is 0.1 mM to 5 mM, a concentration of the sucrose is 50 mM to 800 mM, an amount of the inducibly expressed enzyme product A of the recombinant strain A added is 0.1 mL to 50 mL, and an amount of the inducibly expressed enzyme product B of the recombinant strain B added is 0.1 mL to 50 mL.

10. The method according to claim 7, wherein in the catalytic reaction system, a pH is 5.0 to 8.0, a temperature is 25°C to 60°C, and the reaction is conducted for 5 h to 30 h.

11. A glycosyltransferase UGT91C1 mutant, wherein the glycosyltransferase UGT91C1 mutant is any one selected from the group consisting of following (A) to (C):
(A) a protein produced through any one or more selected from the group consisting of following mutations based on an amino acid sequence shown in SEQ ID NO: 20:
a mutation of a 89th amino acid from N to Y;
a mutation of a 155th amino acid from M to L;
a mutation of a 274th amino acid from S to T; and
a mutation of a 361st amino acid from N to S;
(B) a protein that has an identity of 95% or 98% or more with and a same function as an amino acid sequence defined in the (A); and
(C) a fusion protein produced by linking a tag to a terminus of the protein defined in the (A) or the (B).

12. The glycosyltransferase UGT91C1 mutant according to claim 11, wherein an amino acid sequence of the glycosyltransferase UGT91C1 mutant is shown in SEQ ID NO: 22.

13. A biological material, wherein the biological material comprises any one selected from the group consisting of following:
(A) a gene encoding the glycosyltransferase UGT91C1 mutant according to claim 11 or 12;
(B) a recombinant plasmid carrying the gene in the (A); and
(C) a recombinant cell carrying the recombinant plasmid or the gene encoding the glycosyltransferase UGT91C1 mutant.

14. An enzyme composition, comprising the glycosyltransferase UGT91C1 mutant according to claim 11 or 12 and sucrose synthase AtSUS,
wherein the sucrose synthase AtSUS is following (B1) or (B2): (B1) an amino acid sequence shown in SEQ ID NO: 5; and
(B2) a protein that has an identity of 95% or 98% or more with and a same function as the amino acid sequence defined in the (B1).

15. A complete set of recombinant strains for expressing the enzyme composition according to claim 14, wherein the complete set of recombinant strains comprise a recombinant strain A and a recombinant strain B;
the recombinant strain A comprises the recombinant plasmid according to claim 13; and
the recombinant strain B comprises a recombinant plasmid B, and the recombinant plasmid B is produced by constructing a gene encoding the sucrose synthase AtSUS into a plasmid.

16. The complete set of recombinant strains according to claim 15, wherein host strains comprise, but are not limited to, *Escherichia coli*, *Saccharomyces cerevisiae*, *Pichia pastoris*, or *Corynebacterium glutamicum*.

17. A method for synthesizing a rebaudioside D (RD) under catalysis of a glycosyltransferase UGT91C1 mutant, comprising following steps:
adding RA, UDPG, sucrose, and the enzyme composition according to claim 14 or inducibly expressed enzyme products of the complete set of recombinant strains according to claim 15 to a catalytic reaction system, conducting a reaction, and conducting enzyme inactivation and centrifugation to produce a supernatant comprising the RD.

18. The method according to claim 17, wherein the inducibly expressed enzyme products of the complete set of recombinant strains according to claim 15 comprise an inducibly expressed enzyme product A and an inducibly expressed enzyme product B;
a process for acquiring the inducibly expressed enzyme product A comprises following steps:
inoculating a seed culture of the recombinant strain A according to claim 15 into a medium comprising kanamycin sulfate, and culturing; when OD₆₀₀ of a resulting culture reaches 0.6 to 0.8, adding isopropyl-β-D-thiogalactoside (IPTG), and conducting induction for 8 h to 40 h; conducting centrifugation to produce a first cell pellet, and collecting the first cell pellet; subjecting the first cell pellet to disruption with a lysozyme solution or to ultrasonic disruption, conducting centrifugation to produce a first supernatant, and collecting the first supernatant, which is the inducibly expressed enzyme product A, wherein a final concentration of the kanamycin sulfate is 10 µg/mL to 100 µg/mL and a final concentration of the IPTG is 0.01 mM to 1 mM; and
a process for acquiring the inducibly expressed enzyme product B comprises following steps:
inoculating a seed culture of the recombinant strain B according to claim 15 into a medium comprising kanamycin sulfate, and culturing at 25°C to 40°C and 200 r/min to 300 r/min until OD₆₀₀ of a resulting culture reaches 0.6 to 0.8; adding L-arabinose, and conducting induction for 8 h to 40 h; conducting centrifugation to produce a second cell pellet, and collecting the second cell pellet; subjecting the second cell pellet to disruption with a lysozyme solution or to ultrasonic disruption, conducting centrifugation to produce a second supernatant, and collecting the second supernatant, which is the inducibly expressed enzyme product B, wherein a final concentration of the kanamycin sulfate is 10 µg/mL to 100 µg/mL and a final concentration of the L-arabinose is 0.1 mM to 15 mM.

19. The method according to claim 17 or 18, wherein in the catalytic reaction system, a concentration of the RA is 5 mM to 100 mM, a concentration of the UDPG is 0.1 mM to 5 mM, a concentration of the sucrose is 40 mM to 800 mM, an amount of the inducibly expressed enzyme product A added is 0.1 mL to 50 mL, and an amount of the inducibly expressed enzyme product B added is 0.1 mL to 50 mL.

20. The method according to claim 17 or 18, wherein in the catalytic reaction system, a pH is 5.0 to 8.0, a temperature is 25°C to 60°C, and the reaction is conducted for 5 h to 30 h.

21. A glycosyltransferase UGT76G1 mutant, wherein the glycosyltransferase UGT76G1 mutant is any one selected from the group consisting of following (A) to (C):
(A) a protein produced through any one or more selected from the group consisting of following mutations based on an amino acid sequence shown in SEQ ID NO: 1:
a mutation of an 89th amino acid from M to H;
a mutation of a 380th amino acid from L to M; and
a mutation of a 411th amino acid from A to Y;
(B) a protein that has an identity of 95% or 98% or more with and a same function as an amino acid sequence defined in the (A); and
(C) a fusion protein produced by linking a tag to a terminus of the protein defined in the (A) or the (B).

22. The glycosyltransferase UGT76G1 mutant according to claim 21, wherein an amino acid sequence of the glycosyltransferase UGT76G1 mutant is shown in SEQ ID NO: 30.

23. A biological material, wherein the biological material comprises any one selected from the group consisting of following:
(A) a gene encoding the glycosyltransferase UGT76G1 mutant according to claim 21 or 22;
(B) a recombinant plasmid carrying the gene in the (A); and
(C) a recombinant cell carrying the recombinant plasmid in the (B) or the gene in the (A).

24. An enzyme composition, comprising the glycosyltransferase UGT76G1 mutant according to claim 21 or 22 and sucrose synthase AtSUS,
wherein the sucrose synthase AtSUS is following (B1) or (B2):
(B1) an amino acid sequence shown in SEQ ID NO: 5; and
(B2) a protein that has an identity of 95% or 98% or more with and a same function as the amino acid sequence defined in the (B1).

25. A method for synthesizing a rebaudioside M (RM) under catalysis of a glycosyltransferase UGT76G1 mutant, comprising: with RD, UDPG, and sucrose as substrates, synthesizing the RM under catalysis of the enzyme composition according to claim 24.

26. The method according to claim 25, wherein in a catalytic reaction system, a concentration of the RD is 5 mM to 100 mM, a concentration of the UDPG is 0.1 mM to 5 mM, a concentration of the sucrose is 40 mM to 800 mM, an amount of a glycosyltransferase UGT76G1 mutant solution added is 0.1 mL to 50 mL, and an amount of a sucrose synthase AtSUS solution added is 0.1 mL to 50 mL.

27. The method according to claim 26, wherein a preparation process of the glycosyltransferase UGT76G1 mutant solution comprises following steps:
(1) constructing a gene encoding the glycosyltransferase UGT76G1 mutant into an expression vector to produce a recombinant plasmid A, and transforming the recombinant plasmid A into a host strain to produce a recombinant strain A; and
(2) inoculating a seed culture of the recombinant strain A into a medium comprising kanamycin sulfate, and culturing; when OD₆₀₀ of a resulting culture reaches 0.6 to 0.8, adding L-arabinose, and conducting induction at 25°C to 40°C for 8 h to 40 h; conducting centrifugation, and collecting a resulting cell pellet; subjecting the cell pellet to disruption, and conducting centrifugation to produce a supernatant, which is the glycosyltransferase UGT76G1 mutant solution, wherein an inoculum size is 1 v/v%, a final concentration of the kanamycin sulfate is 10 µg/mL to 100 µg/mL, and a final concentration of the L-arabinose is 0.1 mM to 15 mM.

28. The method according to claim 26, wherein a preparation process of the sucrose synthase AtSUS solution comprises following steps:
(1) constructing a gene encoding the sucrose synthase AtSUS into an expression vector to produce a recombinant plasmid B, and transforming the recombinant plasmid B into a host strain to produce a recombinant strain B; and
(2) inoculating a seed culture of the recombinant strain B into a medium comprising kanamycin sulfate, and culturing; when OD₆₀₀ of a resulting culture reaches 0.6 to 0.8, adding L-arabinose, and conducting induction at 25°C to 40°C for 8 h to 40 h; conducting centrifugation, and collecting a resulting cell pellet; subjecting the cell pellet to disruption, and conducting centrifugation to produce a supernatant, which is the sucrose synthase AtSUS solution, wherein an inoculum size is 1 v/v%, a final concentration of the kanamycin sulfate is 10 µg/mL to 100 µg/mL, and a final concentration of the L-arabinose is 0.1 mM to 15 mM.

29. The method according to claim 27 or 28, wherein the host strain comprises, but is not limited to, *Escherichia coli*, *Saccharomyces cerevisiae*, *Pichia pastoris*, or *Corynebacterium glutamicum*.

30. The method according to claim 25 or 26, wherein in the catalytic reaction system, a pH is 5.0 to 8.0, a temperature is 25°C to 60°C, and a reaction is conducted for 5 h to 30 h.
